# EUROPEAN PATENT APPLICATION

(11) **EP 4 144 719 A1**
(43) Date of publication of application: **08.03.2023**
(21) Application number: 21797674.5
(22) Date of filing: 27.04.2021
(51) Int. Cl.: C07C 231/06, C07C 231/02, C07C 233/04, C07C 233/05, C07C 69/00, A01N 37/02

(54) **ARTHROPOD REPELLENTS OBTAINED BY CHEMICALLY CONVERTING LACTIC ACID, LACTATES OR OTHER LACTIC ACID DERIVATIVES**

(30) Priority: 28.04.2020 BR 102020008433
(71) Applicant: Universidade Federal Do Paraná, 80010-200 Curitiba (BR)
(72) Inventor: DE ASSIS MARQUES, Francisco, 83030-240 S. José Pinhais (BR); LAMEIRO DE NORONHA SALES MAIA, Beatriz Helena, 81530-560 Curitiba (BR); ANNIES, Vinicius, 80730-000 Curitiba (BR); GONÇALVES LARA, Renata, 81531-980 Curitiba (BR); DA SILVA SANTOS, Nayana Cristina, 82960-010 Curitiba (BR); BAIAK, Rita De Cássia, 84603-304 União da Vitória (BR)
(74) Representative: Clarke Modet & Co.
(86) International application number: PCT/BR2021/050176
(87) International publication number: WO 2021/217227

(57) **Abstract**

The present invention belongs to the fields of green chemistry and human health, more specifically, it describes the production of a high efficiency arthropod repellent by chemically transforming a substance used by mosquitoes as an attractant, the lactic acid, as well as lactates, in this case (S)-ethyl lactate. Lactic acid is usually found in the perspiration and body odor of all warm-blooded animals and is an essential attractant for *A. aegypti* females. The present invention is based on the principle of using the structure of said substance, which already interacts with mosquito receptors and acts as an attractant, to modify it. To this end, the hydroxyl functional group of lactic acid must be transformed into esters, and the carboxylic acid functional group into amides, since esters and amides are functional groups already present in other repellents, such as in the structure of DEET, IR 3535 and dimethyl phthalate. As for the lactates, in the case of (S)-ethyl lactate, the ester functional group must undergo a reaction in which the acyl functional group is substituted by an amine of interest and the hydroxyl functional group must be acylated. All the steps used in the synthesis of molecules having a repellent effect, both from lactic acid and lactates, are widely described in the literature. In other words, by using a structure that already interacts with mosquito receptors and modifying same by transforming its chemical functional groups into other functional groups already found in other repellents, it is possible to transform the structure of an attractant, or a derivative thereof, into a high efficiency repellent.

## Description

The present invention belongs to the fields of green chemistry and human health, more specifically, it describes the use of the natural mosquito attractant lactic acid, and its derivatives such as lactates, chemically modified, to constitute active ingredients for arthropod repellents.

### STATE OF THE ART

Repellents can be defined as volatile chemicals that induce arthropods to move towards the opposite direction to previously treated surfaces, thereby repelling them. These products come to consumers in a multitude of formulations and applications, such as aerosols, creams, sprays, screens, or impregnated clothing.

Ideally, a repellent should have the following characteristics: has long-lasting effectiveness against a wide variety of arthropods; is harmless to humans after application to skin or clothing; is odorless or smell pleasant; has no oily sensation when applied to the skin, and resists removal by rubbing, washing or sweating; is inert when in contact with commonly used plastics (such as eyeglass frames, pens, etc.); is chemically stable and economically viable for widespread use.

All repellents exhibit some degree of volatility, and the boiling point of substances with repellent action is usually between 230 °C and 260 °C. Compounds with a lower boiling point dissipate more easily and have a reduced repellency time, and compounds with higher boiling points are ineffective as they fail to volatilize at an effective rate to exert the desired effect.

Most repellents, once applied to the skin or clothing in a small area around the application, work by producing a vapor layer that creates an unpleasant or offensive surface for mosquitoes, but there are also repellents that neutralize the compounds which attract arthropods toward humans, by acting as a camouflage.

The repellency time depends on several factors and each compound has a different intrinsic protection that diverges among mosquito species. Upon application, there is a period when the substance repels all mosquitoes and no landing or biting is observed, followed by a period when the substance begins to lose some of its effects, allowing the mosquitoes to land on the treated skin. When the repellent completely loses its effectiveness is when the biting occurs.

The use of repellents dates back to ancient times, when plants were burned so as to use the smoke produced, or oils were extracted to be used in insect repellency. Countless plants and their derivatives, in particular essential oils, have already been investigated and described as potential natural sources for insect repellents.

Repellents from natural sources currently available on the market usually contain substances obtained from citronella (*Cymbopogonnardus)*, eucalyptus (*Eucalyptusmaculata)*, geranium (*Pelargonium reniforme)*, lemon grass (*Cymbopogoncitratus)*, among others. Although natural repellents have a shortlasting effect compared to synthetic ones, their toxicity is usually lower, which justifies the efforts made to seek compounds with repellent activity in plant extracts.

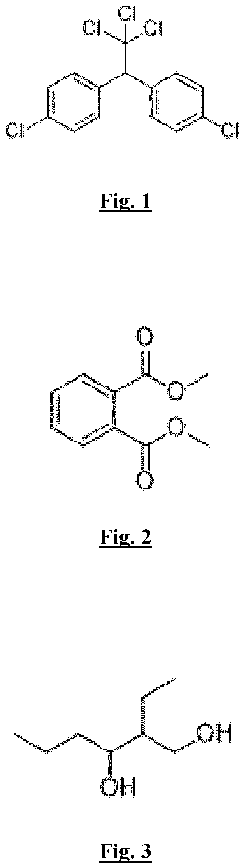

As for synthetically obtained repellents, studies have been started and several compounds were tested in the period of the Second World War. Until 1942, the compounds DDT (dichlorodiphenyltrichloroethane), DMP (dimethyl phthalate) and Rutgers 612 (2-ethyl-1 ,3-hexanediol), whose structures are shown in Figures 1, 2 and 3, respectively, were approved to be used by the American army, showing a two-hour repellency. However, its uses were later vetoed due to toxicity observations.

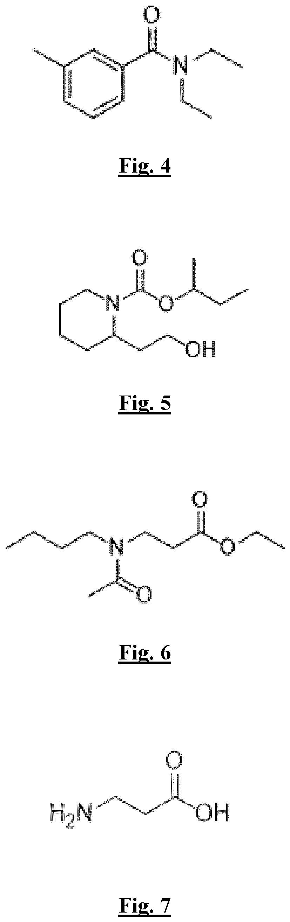

In 1946, DEET (N,N-diethyl-3-methylbenzamide) began to be used, a repellent deemed as the reference standard up to this day and which, together with picaridin and IR 3535, are the most recommended and sold ones. DEET, represented in Figure 4, is one of the oldest and most used repellent active ingredient, which was first evaluated in 1944 (against *Aedes aegypti)* and patented by the U.S. Army in 1946. After further studies in 1952, it was finally launched on the market in 1954 and became widely used in 1957. There are currently about 140 products containing DEET registered with the U.S. Environmental Protection Agency, being manufactured by 40 companies.

Said repellent acts by decreasing the response of insects attracted by lactic acid (released through perspiration), which can classify it as a behavioral inhibitor that reduces attraction rather than activating a repelling behavior. The non-patent literature LEGEAY, S.; CLERE, N.; APAIRE-MARCHAIS, V.; FAURE, S.; LAPIED, B. Unusual modes of action of the repellent DEET in insects highlight some human side effects. European Journal of Pharmacology, v. 825, p. 92-98, 2018, discloses studies carried out on different species of arthropods indicating that DEET acts as a sensory stimulus and can be detected by the olfactory receptor neurons present in the antennae of different species, making this repellent efficient against a wide variety of insects.

Its major advantage is its low cost and long-lasting effect, which can last more than 6 hours at a concentration of 20% to 30% of the compound. However, among the currently available repellents, its toxicity is the highest. It has been described that DEET can cause damage to synthetic clothing fibers, plastics, and acrylics, and it is not recommended for use by pregnant women or children under two years of age, as it causes various forms of skin allergies.

Icaridin, also known as picaridin, is an active ingredient derived from pepper whose structure is depicted in Figure 5. It is a widely used and effective repellent against mosquitoes, flies, ticks, etc. As it evaporates more slowly from the skin, it offers a longer repellency time than DEET, in addition to being more effective against mosquitoes of the genus *Aedes.* Its minimum concentration for repellent action starts at 5% and at a concentration of 20%, it offers protection of up to 10 hours. It is an odorless, non-oily repellent, does not cause damage to plastics and acrylics and the use on children under six months is allowed. Nevertheless, it can cause damage to some animal leather clothing and materials. Icaridin has two stereocenters and is marketed as a mixture of stereoisomers.

Available since 1980, IR 3535 (ethyl butylacetylaminopropionate), Figure 6, has its chemical structure based on a natural substance (β-alanine), Figure 7. Said component is effective against mosquitoes, flies, ticks, lice, wasps, and bees. Its effectiveness is slightly lower compared to DEET, if used in a concentration above 10%.

The mechanism of action of IR 3535 consists of forming a vapor barrier that prevents the contact of insects with the skin due to the offensive odor that the substance poses thereto, although it is odorless to humans. Despite being effective for a shorter time, said compound has a much lower toxicological profile and can be used by young children and pregnant women.

It that can be observed that the structures of DEET and IR3535 both exhibit an amide group, while IR 3535 also bears an ester group, as can also be seen in the structure of dimethyl phthalate.

Some works have already explored the influence of these groups on the repellency property provided by these compounds, consisting in the evaluation of repellents' analogues commercially used.

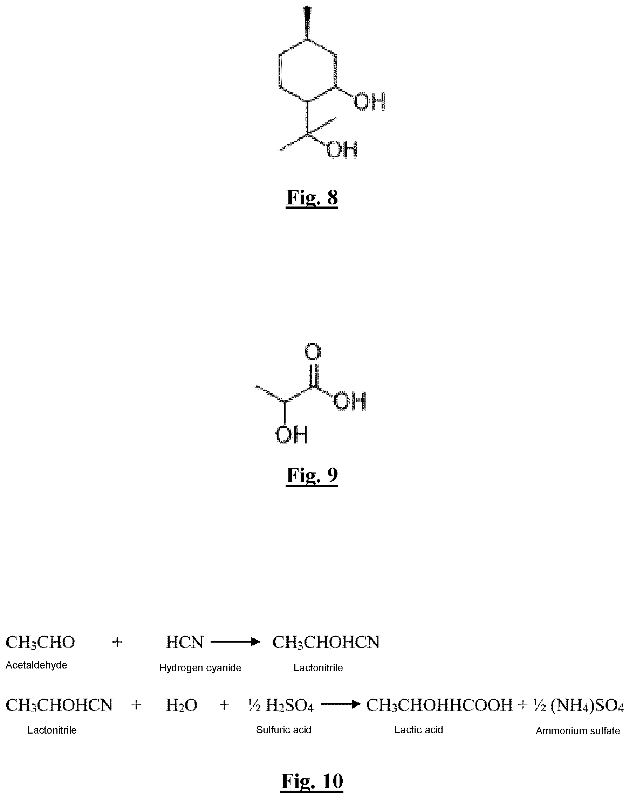

The p-menthane-3,8-diol, depicted in Figure 8, is the active ingredient derived from a natural product most used worldwide, and was isolated from *Corymbiacitriodora,* also known as lemon eucalyptus or fragrant eucalyptus.

Patent documents comprising the state of the art, such as JP2000119109A, CA2648270A1, JP2010116340A and CN110538082A, despite featuring different insecticidal composition solutions, even including lactic acid, fail to provide a method of synthesizing a repellent from lactic acid or lactate and its resulting product under the specific conditions of the present invention. Thus, the patent documents found do not reveal the transformation of the chemical functional groups of lactic acid, or derivatives such as lactates, into other functional groups, making them repellent.

Patent NL8000126, "New alpha-acyloxyacetamides, process for their preparation and use thereof as intermediates" filed on July 14, 1980, refers to derivatives of acyloxyacetamides of the general formula I, involving the preparation of amides and their pharmacological properties, acting as broncholytics.

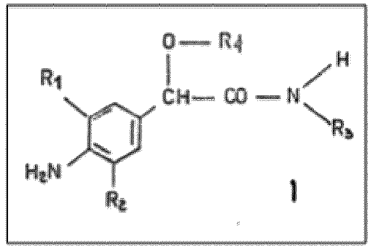

Patent US4166123, "Acylaminopyrazoles", filed on August 28, 1979, refers to derivatives of the general formula I and their uses for the treatment of asthma.

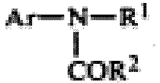

Document "Phenyl alpha-acyloxyacetamide derivatives and their therapeutic uses", filed on May 21, 1985, refers to the use of substances of general formula I for the treatment of dermatological diseases.

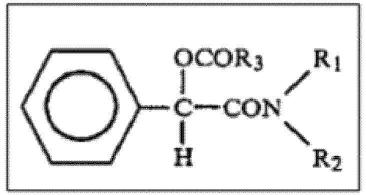

Document titled "INSECT REPELLENTS. III. N,N-DIETHYLAMIDES", published in 1954 (McCabe, ET et al.; J. Org. Chem.1954, 19, 4, 493-498), discusses the structural correlation and repellent activity and mentions that the chances of finding substances with repellent activities among esters are greater than among hydrocarbons. It also describes that among the various functional groups studied, certain diols and the N,N-dialkylamides, especially diethylamides, are promising as a source of mosquito repellents. Furthermore, the document describes the process of synthesizing repellents from organic acids by acylation process.

The non-patent literature "L-Lactic Acids to Mosquito (Diptera: Culicidae) Repellent on Human and Mouse Skin", published in 2001 (Shirai, Y. et al., J. Med. Entomol., 38, 1, 51-54), reveals a study in which the repellent effect of lactic acid is evaluated as a function of its concentration in different formulations.

One can find on the market products with repellent action in different forms of presentation such as lotion, gel, spray, whether the active ingredient has been encapsulated or not.

It is also possible to find indoor repellents, known as repellents for internal environments, and outdoor repellents.

Products with mosquito repellent action can also be repellent to other arthropods such as wasps, ticks, lice, and fleas.

### TECHNICAL PROBLEM

The great challenge of a repellent product lies in providing a low toxicological profile, a satisfactory performance time and not causing several damages, such as to materials, fabrics, and animal leather clothing.

### TECHNICAL SOLUTION

Lactic acid is normally present in the perspiration and body odor of all warm-blooded animals and is an essential attractant for *A. aegypti* females.

The present invention is based on the principle of using the structure of said substance, which already interacts with mosquito receptors and acts as an attractant, to chemically modify it. To this end, the hydroxyl functional group of lactic acid must be transformed into esters, and the carboxylic acid functional group into amides, since esters and amides are functional groups already present in other repellents, such as in the structure of DEET, IR 3535 and dimethyl phthalate. In other words, by using a structure that already interacts with mosquito receptors and modifying it by the transformation of its chemical functional groups into other functional groups already present in other repellents, after evaluating the role that some esters and amides had in the activity of the prepared derivatives, it was possible to transform the structure of an attractant into a repellent.

Through the synthesis of a series of new substances from (S)-lactic acid or from (S)-ethyl lactate according to the present invention, it is possible to select the best combinations of ester and amide to potentiate the repellent effect of the prepared substance.

Molecules with repellent action, resulting from the present invention, can be formulated and have different forms of presentation such as lotion, gel, spray, whether the active ingredient has been encapsulated or not.

It is also possible that repellents containing the substances resulting from the present invention are used indoors, known as repellents for internal environments, and outdoors.

The products containing the active ingredients described in this invention, as is the case with other repellents already known, may have repellent action against mosquitoes as well as other arthropods such as wasps, ticks, lice, and fleas.

By using the substances 02, 04, 05, 08, 10 and 11, shown in Table 1, as a composition active ingredient, the repellents prepared can have several possible forms of presentation, such as repellent body lotion, repellent body spray, repellent body cream gel, repellent sunscreen lotion, repellent sunscreen spray, repellent sunscreen aerosol, environmental repellent capsule, repellent liquid for electric sprinkler, microcapsules and/or capsules using the active ingredient as a repellent, repellent powder for indoor use, repellent powder for outdoor use, indoor and/or outdoor repellent gel, repellent wall paint, repellent coating putty. These forms of presentation may be used in animals and/or agricultural environment

### DIFFERENTIAL ASPECTS OF THE INVENTION

A differentiating feature of this approach is that both (S)-lactic acid and (S)-ethyl lactate are biodegradable, produced on a large scale, and have low toxicity and low cost.

The use of (S)-ethyl lactate as a starting material gives the process an advantage since the synthesis of the amide of interest can be performed with no solvent. Furthermore, the second step of the synthesis can be optimized by using a heterogeneous catalyst, and also performed with no solvent. The use of microwaves drastically reduces the time required for this reaction to occur.

When synthesizing the repellent from (S)-lactic acid on a large scale, the methodology to prepare the amide functional group present in the repellent would require a reactor with inert material, since it was made with thionyl chloride (SOCl₂), a highly reactive and toxic reagent.

*(S)*-ethyl lactate is produced on a large scale and the fact that the repellent synthesized from this enantiomer is more efficient than that obtained from the racemic mixture prepared in the laboratory, makes it quite attractive from a commercial point of view, as it should give the repellent a more affordable price.

Therefore, the synthesis route of the repellent of the present invention, that is, from ethyl lactate, is more interesting to be applied on a large scale, considering that the two steps could be carried out using less toxic reagents and without requiring the use of solvents.

### BRIEF DESCRIPTION OF THE FIGURES

The description as follows is not limited to the drawings or components cited, with reference to the following illustrations referenced below.
Figure 1 represents the structure of the substance DDT.
Figure 2 represents the structure of the substance dimethyl phthalate.
Figure 3 represents the structure of the substance 2-ethyl-1,3-hexanediol.
Figure 4 represents the structure of DEET repellent.
Figure 5 represents the structure of Icaridin repellent.
Figure 6 represents the structure of the IR3535 repellent.
Figure 7 represents the structure of β-alanine.
Figure 8 represents the structure of *p*-menthane-3,8-diol repellent.
Figure 9 represents the structure of lactic acid.
Figure 10 represents the chemical synthesis of lactic acid through the hydrolysis of lactonitrile.
Figure 11 represents the synthesis of *(S)*-ethyl lactate.
Figure 12 represents the proposed synthesis of candidate repellent molecules from *(S)*-lactic acid.
Figure 13 represents the proposed synthesis of candidate repellent molecules from *(S)*-ethyl lactate.
Figure 14 represents the hydroxy amide oxidation reaction.
Figure 15 represents the synthesis of the repellent racemic mixture.
Figure 16 represents the hand positioning in the cage during the repellency tests performed

### DETAILED DESCRIPTION

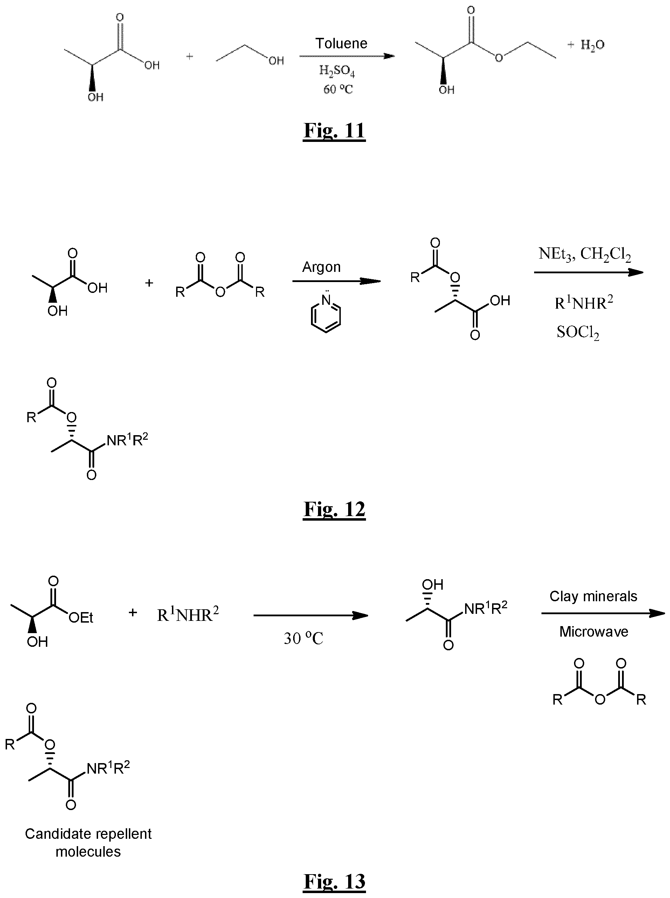

The present invention describes the production of a high-efficiency arthropod repellent by chemically transforming a substance used by mosquitoes as an attractant, the lactic acid, as well as lactates, in which the synthesis route from *(S)*-lactic acid involves the transformation of the hydroxyl functional group into esters through acylation reactions, and the transformation of the carboxyl functional group into amides, Figure 12, using, in both cases, reactions widely known and described in the literature.

In the first step of the repellent synthesis from *(S)*-lactic acid, the syntheses of structures with potential to be repellents are carried out, starting the process with an acylation reaction of the hydroxyl functional group using pyridine and different acylating agents. The second synthesis step, starting from *(S)*-lactic acid, initially involves the conversion of the carboxyl group into an acyl halide, through the reaction with thionyl chloride (SOCl₂), which previously reacts with the amines of interest, as shown in Figure 12. This reaction with thionyl chloride has disadvantages because this reagent is toxic, very reactive and requires more controlled reaction conditions.

Another synthesis route used to prepare this repellent uses lactates as starting material, in this case *(S)*-ethyl lactate, which gives an advantage to the process since the first step in the synthesis of the amides of interest, which consists of an acyl substitution reaction, is carried out without requiring the use of solvents and thionyl chloride, thus obtaining an improvement in the synthesis process. This synthesis proposal is represented in Figure 13.

In the second step of the synthesis from lactate, the acylation reaction of the hydroxyl functional group is performed by reactions commonly used for this purpose. This step was optimized using heterogeneous catalyst in the absence of solvent. The use of microwaves, setting the temperature at 90°C, reduces the reaction time from 6 hours to 15 minutes, which makes the process attractive for application on an industrial scale. Therefore, it is possible to synthesize several molecules.

Table 1 shows substances synthesized from *(S)*-ethyl lactate or *(S)*-lactic acid with the respective repellency times of solutions of 15 to 20% in ethanol.

| Table 1 | | | |
|---|---|---|---|
| Input | From | Substance | Repellency |
| 1 | *(S)*-ethyl lactate with 100% optical purity | 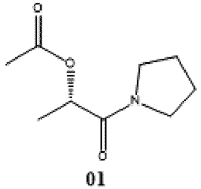 | 4 hours and 10 minutes |
| 2 | *(S)*-lactic acid with 85% optical purity | 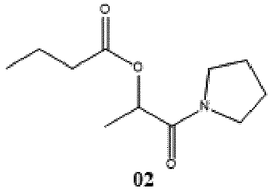 | > 10 hours |
| 3 | *(S)*-ethyl lactate with 100% optical purity | 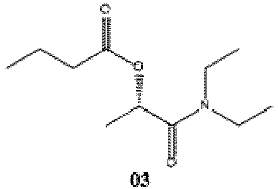 | 2 hours |
| 4 | *(S)*-ethyl lactate with 100% optical purity | 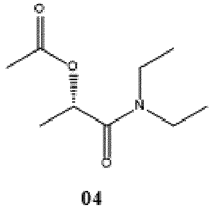 | 5 hours |
| 5 | *(S)*-ethyl lactate with 100% optical purity | | > 10 hours |
| | | 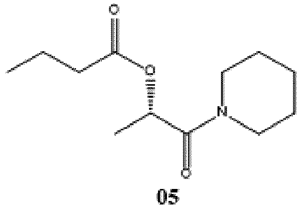 | |
| 6 | *(S)*-ethyl lactate with 100% optical purity | 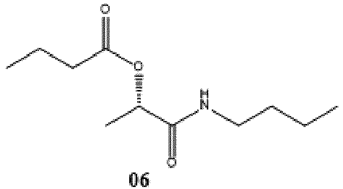 | 2 hours and 30 minutes |
| 7 | *(S)*-lactic acid with 85% optical purity | 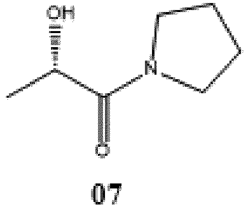 | 0 hours |
| 8 | *(S)*-ethyl lactate with 100% optical purity | 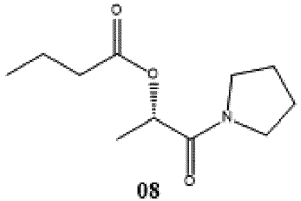 | > 12 hours |
| 9 | *(S)*-ethyl lactate with 100% optical purity | 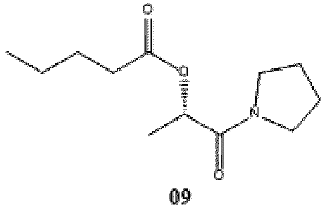 | 3 hours and 20 minutes |
| 10 | *(S)*-ethyl lactate with 100% optical purity | 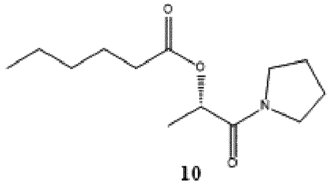 | 8 hours and 50 minutes |
| 11 | racemic repellent mixture | 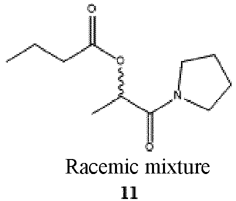 | 10 hours |

As shown in Table 1, substance 8, prepared with four carbon atoms in the acyl portion of the ester and containing the five-membered cyclic amine, showed the best result in terms of repellency time.

In order to evaluate the influence of the stereochemistry of the stereocenter present in this structure on the repellent activity of this substance, having the enantiomerically pure hydroxyamide obtained from (S)-ethyl lactate, the racemic mixture is obtained, to better understand the effect of stereochemistry on the substance repellency.

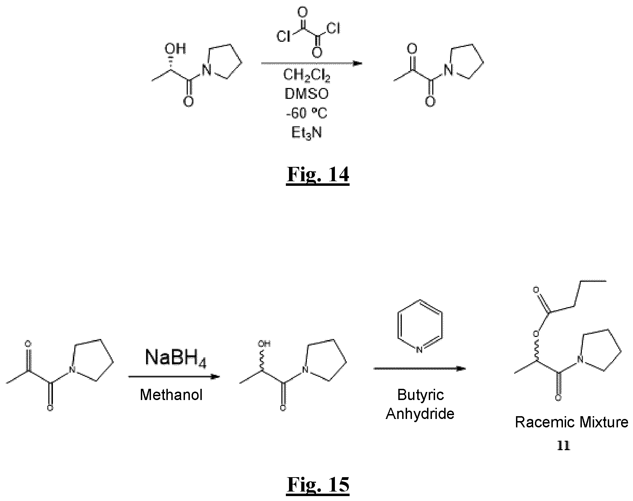

The racemate production consists of using the Swern oxidation to oxidize the alcohol to the corresponding ketone, as shown in Figure 14, thereby obtaining a molecule lacking a stereocenter.

In the presence of the ketone, a reduction reaction is carried out with sodium borohydride, as shown in Figure 15. The use of this reagent ensures that the reaction is chemoselective, whereby only the ketone is reduced, resulting in the production of the alcohol in its racemic form. This alcohol is then acylated under the described conditions. This series of reactions, well known in the chemical literature, results in the synthesis of the two enantiomers of the compound, as confirmed by polarimeter analysis.

As shown in Table 1, the result of the repellency obtained using the mixture of the two stereoisomers, input 11, it can be observed that although the repellency decreases from more than 12 hours to approximately 10 hours, the racemic mixture is also effective.

The synthesis of candidate repellent molecules by means of (S)-lactic acid are described below through procedures employed for the synthesis of candidate repellent molecules, including molecule 2 of Table 1, from (S)-lactic acid with 85% optical purity.

In the case of the lactic acid acylation reaction, lactic acid (0.84 g, 8 mmol) and pyridine (5 mL) are added in a reaction vessel under an inert atmosphere. Afterward, the anhydride selected for each reaction (12 mmol) is added, allowing it to stand for 24 hours at room temperature. Next, ice water (20 ml) is added, and the reaction mixture is acidified to pH 1-2 with 36.5% hydrochloric acid; the phases are separated and then the aqueous solution is extracted with ethyl acetate (3 x 20 mL). The organic phases are combined and washed with saturated sodium bicarbonate solution (1 × 20 ml) and with saturated sodium chloride solution (1 × 20 ml). After being dried over anhydrous sodium sulfate, the organic phase is filtered, and the solvent removed under reduced pressure. The products obtained are then purified by column chromatography using a gradient of hexane and ethyl acetate as eluent. The yield of ester ranges from 60 to 70%.

As for the amidation reaction of the acylated (S)-lactic acid derivative, the acylated lactic acid derivative product (5.1 mmol) is added into a flask and placed under inert atmosphere. Dichloromethane (15 mL) is then added as a reaction solvent. Next, triethylamine (1.52 g, 15 mmol), the selected amine (5.1 mmol) and lastly, thionyl chloride (6 mmol), are added dropwise. The reaction is then left at room temperature for 24 hours. Afterwards, the reaction mixture is transferred to a separatory funnel and washed with 1 mol L⁻¹ hydrochloric acid solution (2 × 20 mL) and once with 1 mol L⁻¹ sodium hydroxide solution (1 × 20 mL). Then the aqueous phase is extracted with dichloromethane (3 × 20 mL). The organic phases are combined and dried over anhydrous sodium sulfate, followed by filtration and evaporation of the solvent under reduced pressure. The reaction products are then purified via column chromatography using a gradient of hexane and ethyl acetate as eluent. The yields of the products after purification ranged from 65 to 85%.

The synthesis of repellent candidate molecules by means of lactate, preferably *(S)*-ethyl lactate, are described below by procedures employed for the synthesis of molecule 8 in Table 1, as a standard procedure used for the synthesis of all repellent candidate molecules from *(S)*-ethyl lactate.

As for the acyl substitution reaction of *(S)*-ethyl lactate, pyrrolidine (1.81 g, 30 mmol) is added into a flask and, on an ice bath, the *(S)*-ethyl lactate (Aldrich) (2.416 g, 20 mmol) is added. After the addition of the reagents, the flask is heated until it reaches a temperature of 30 °C. The reaction is left for 72 hours. Residual amine and alcohol are distilled off under reduced pressure. The reaction product is then purified via column chromatography using a gradient of hexane and ethyl acetate as eluent. Product yield is 91%.

As for the amide acylation reaction, the amide obtained in the previous step (2.99 g, 20.9 mmol) and the heterogeneous catalyst (5 mol %) are added into a reaction vessel under an inert atmosphere. Then, butanoic anhydride (31.3 mmol) is added, and the reaction is carried out under microwave, setting the temperature at 90°C for 15 minutes. After this period, the catalyst is filtered, and the contents of the reaction flask are transferred to a separatory funnel containing ethyl ether (20mL). The organic phase is washed with saturated sodium bicarbonate solution (3 × 20 ml) and with saturated sodium chloride solution (1 × 20 ml). After being dried over anhydrous sodium sulfate, the organic phase is filtered, and the solvent was removed under reduced pressure. The product obtained is then purified by column chromatography using a gradient of hexane and ethyl acetate as eluent, obtaining the molecule 8 in Table 1 with 60% yield.

As for the synthesis of the racemic mixture represented by structure 11, anhydrous dichloromethane (2 mL) and oxalyl chloride (1.20 mL, 13.7 mmol) were added into a flask under an inert atmosphere. The system was cooled to - 60 °C and then dimethyl sulfoxide (2.08 mL, 28.56 mmol) was added and allowed to stir for five minutes. After this period, the amide (1.708 g, 12 mmol) dissolved in dichloromethane (2 mL) was added and the mixture was stirred for twenty minutes, maintaining the temperature at -60 °C. Upon completion, triethylamine (8.15 mL, 17.73 mmol) was added, and the mixture was stirred for five minutes. Finally, it was allowed to reach room temperature and water (10 mL) was added. The reaction mixture was extracted with dichloromethane (3x of 30 mL). The organic phase was washed with saturated sodium chloride solution (2x of 30 mL), dried over anhydrous Na₂SO₄, followed by filtration and evaporation of the solvent under reduced pressure, resulting in the production of the ketone shown in Figure 14, which was immediately reduced.

For the reduction of the ketone, the same was added into a flask, at room temperature, and methanol (10 mL), the ketone obtained in the previous reaction (1.005 g, 6.98 mmol) and sodium borohydride (0.265 g, 7 mmol) were added and allowed to stir for 24 h. After the reaction was completed, water (1 ml) was added, and methanol was evaporated under reduced pressure. The reaction mixture was then neutralized to pH 6 with hydrochloric acid solution (1 mol L⁻¹), the phases were separated, and the aqueous phase was extracted with dichloromethane (3 x 5 ml). The organic phases were combined and dried over anhydrous Na₂SO₄, followed by filtration and evaporation of the solvent under reduced pressure. The material obtained in this reaction was subjected to the acylation reaction as described in the procedure specified in paragraph [069], whereby obtaining substance 11.

The spectral data of the substances synthesized of Table 1 are described below in Tables 2 to 10.

| Table 2 |
|---|
| Input 1: (S)-1-oxo-1-(pyrrolidin-1-yl)propan-2-yl acetate |
| 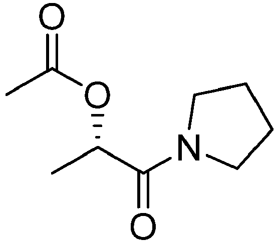 |
| MS (m/z) (%): M⁺¹ 186, 125 (23), 98 (100), 70 (23), 55 (72), 43 (47). |
| ¹H NMR (200 MHz, CDCl₃): δ 5.19 (q, 1H), 3.72-3.32 (m, 4H), 2.13 (s, 3H), 2.05-1.83 (m, 4H), 1.44 (d, 3H). |
| ¹³C NMR (50 MHz, CDCl₃): δ 170,5, 168,7, 68,2, 45,9, 26,0, 23,8, 20,6, 16,3. |

| Table 3 |
|---|
| Inputs 2, 8 and 11: *(S)*-1-oxo-1(pyrrolidin-1-yl)propan-2-yl butyrate |
| 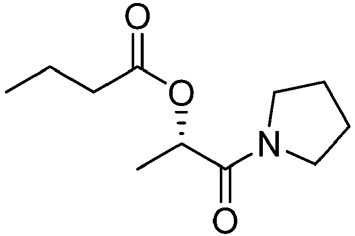 |
| MS (m/z) (%): M⁺¹ 214 (0.23), 125 (30), 98 (100), 71 (43), 55 (32), 43 (17). ¹H NMR (200 MHz, CDCl₃): δ 5.20 (q, 1H), 3.72-3.32 (m, 4H), 2.38 (t, 2H), 2.05-1.82 (m, 4H), 1.76-1.57 (m, 2H), 1.43 (d, 3H), 0.95 (t, 3H). |
| ¹³C NMR (50 MHz, CDCl₃): δ 173.3, 168.8, 68.0, 45.9, 35.7, 26.1, 23.8, 18.2, 16.4, 13.5. |

| Table 4 |
|---|
| Input 3: *(S)*-1-(diethylamino)-1-oxopropan-2-yl butyrate |
| 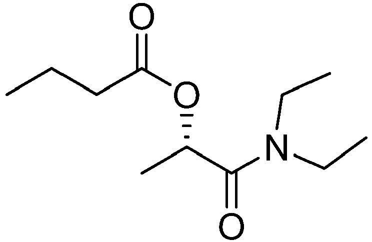 |
| MS (m/z) (%): M⁺¹ 216 (0.19) 127 (19), 100 (100), 72 (57), 43 (14). |
| ¹H NMR (200 MHz, CDCl₃): δ 5.33 (q, 1H), 3.54-3.21 (m, 4H), 2.50-2.25 (m, 2H), 1.77-1.54 (m, 3H), 1.44 (d, 3H), 1.27 (t, 3H), 1.12 (t, 3H), 0.96 (t, 3H). |
| ¹³C NMR (50 MHz, CDCl₃): δ 169.6, 168.9, 66.7, 41.6, 40.5, 35.8, 18.2, 17.2, 14.2, 13.6, 12.8. |

| Table 5 |
|---|
| Input 4: *(S)*-1-(diethylamino)-1-oxopropan-2-yl acetate |
| 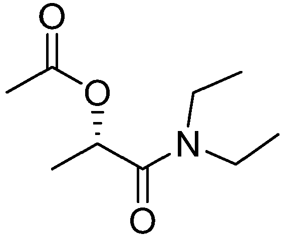 |
| MS (m/z) (%): M⁺¹ 188 (0.45), 127 (25), 100 (100), 87(10), 72 (99), 43 (33) ¹H NMR (200 MHz, CDCl₃): δ 5.31 (q, 1H), 3.57-3.21 (m, 4H), 2.12 (s, 3H), 1.44 (d, 3H), 1.23 (s, 3H), 1.13 (s, 3H). |
| ¹³C NMR (50 MHz, CDCl₃): δ 170.6, 169.7, 66.8, 41.6, 40.6, 20.7, 17.1, 14.1, 12.7. |

| Table 6 |
|---|
| Input 5: *(S)*-1-oxo-1-(piperidin-1-yl)propan-2-yl butyrate |
| 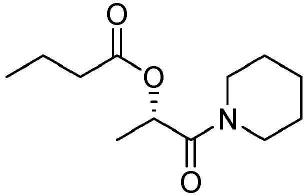 |
| MS (m/z) (%): M⁺¹ 228 (0.10), 139 (19), 112 (100), 84 (15), 71 (31), 69 (44), 43 (21). |
| ¹H NMR (200 MHz, CDCl₃): δ 5.43 (q, 1H), 3.71-3.76 (m, 4H), 2.37 (dt, 2H), 1.76-1.56 (m, 9H), 1.42 (d, 3H), 0.96 (t, 3H). |
| ¹³C NMR (50 MHz, CDCl₃): δ 173.1, 168.4, 66.5, 46.3, 43.2, 35.8, 26.2, 25.4, 24.4, 18.2, 16.8, 13.5. |

| Table 7 |
|---|
| Input 6: *(S)*-1-(butylamino)-1-oxopropan-2-yl butyrate |
| 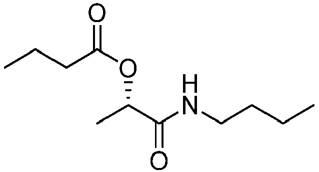 |
| MS (m/z) (%): M⁺¹ 216 (1.24), 143 (20), 116 (34), 100 (51), 88 (49), 71 (100), 57 (46), 43 (43). |
| ¹H NMR (200 MHz, CDCl₃): δ 6.20 (sl, 1H), 5.20 (q, 1H), 3.26 (q, 2H), 2.37 (t, 2H), 1,68 (q, 2H), 1.45 (d, 5H), 1.39-1.22 (m, 2H), 1.00-0.88 (m, 6H) |
| ¹³C NMR (50 MHz, CDCl₃): δ 172.0, 170.2, 70.3, 38.8, 36.1, 31.5, 19.9, 18.3, 17.8, 13.6, 13.4. |

| Table 8 |
|---|
| Input 9: *(S)*-1-oxo-1-(pyrrolidin-1-yl)propan-2-yl pentanoate |
| 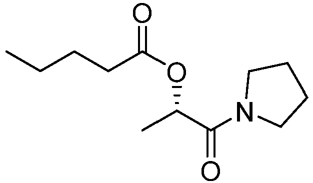 |
| MS (m/z) (%): (M⁺¹ 228 0.19%), 125 (21), 98 (100), 85 (25), 70 (16), 57 (23), 55 (32). |
| ¹H NMR (200 MHz, CDCl₃): δ 5.19 (q, 1H), 3.71-3.31 (m, 4H), 2.38 (dt, 2H), 2.06-1.81 (m, 4H), 1.69-1.54 (m, 2H), 1.42 (d, 3H), 1.37-1.22 (m, 2H), 0.90 (t, 3H). |
| ¹³C NMR (50 MHz, CDCl₃): δ 173.5, 168.8, 68.0, 45.9, 33.5, 26.7, 26.1, 23.8, 22.1, 16.3, 13.5. |

| Table 9 |
|---|
| Input 10: *(S)*-1-oxo-1-(pyrrolidin-1-yl)propan-2-yl hexanoate |
| 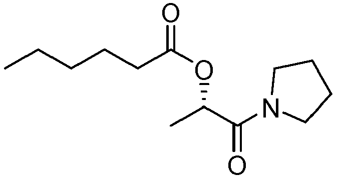 |
| MS (m/z) (%): (M⁺¹ 242 0.23%), 125 (25), 99 (30), 98 (100), 70 (20), 55 (34), 43 (17). |
| ¹H NMR (200 MHz, CDCl₃): δ 5.19 (q, 1H), 3.72-3.32 (m, 4H), 2.42-2.34 (dt, 2H), 2.07-1.82 (m, 5H), 1.43 (d, 3H), 1.37-1.22 (m, 5H), 0.89 (t, 3H). |
| ¹³C NMR (50 MHz, CDCl₃): δ 173.5, 168.88, 68.0, 46.0, 33.8, 31.2, 26.1, 24.3, 23.8, 22.2, 16.4, 13.8. |

Repellency tests can be carried out in the laboratory, at a temperature of 20 °C ± 2 °C and a relative humidity of 50 to 60%. Temperature and relative humidity data should be monitored and recorded using a digital thermohygrometer during the experiments.

To perform the tests, there were used wooden cages with screens on the sides and glass on the upper face measuring 30 cm x 30 cm x 30 cm containing 20 female *A. aegypti* mosquitoes, all mated with no previous blood meal. The tests must be carried out during the daytime, respecting the preference for the mosquito's hematophagy activity.

For the tests, one of the hands of the analyzer must be sanitized with neutral soap and then the upper, lower, lateral face and the region between the fingers must be treated with repellent solutions in ethanol, in order for the compounds to have their repellency activities evaluated. Regarding the tests of the possible repellents provided herein, there were prepared solutions diluted in ethanol, with a concentration of 85 µL mL⁻¹ in a total volume of 1.0 mL.

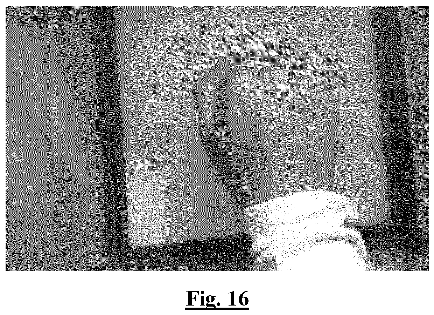

The closed hand is then introduced into the cage to perform the tests, as shown in Figure 16, wherein the analyzer is seated, that is, in a resting position, with no physical effort being performed prior to the test realization. When the first bite occurs, the experiment is interrupted, and the time is recorded by a digital stopwatch.

## Claims

1. Arthropod repellents **characterized by** being synthesized from lactic acid, lactates, or other lactic acid derivatives.

2. Compound synthesized according to claim 1, **characterized by** formula (02): 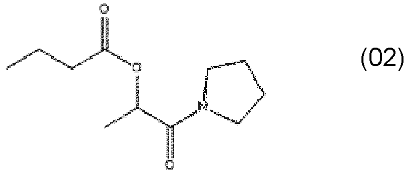 wherein formula (02) represents a (S)-1-oxo-1(pyrrolidin-1-yl)propan-2-yl butyrate obtained from lactic acid with 85% optical purity.

3. Compound synthesized according to claim 1, **characterized by** formula (04): 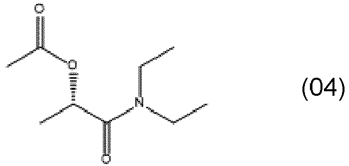 wherein formula (04) represents a (*S*)-1-(diethylamino)-1-oxopropan-2-yl acetate.

4. Compound synthesized according to claim 1, **characterized by** formula (05): 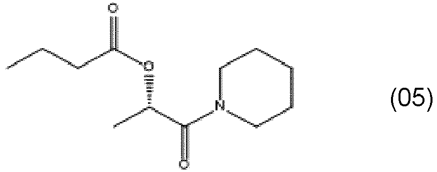 wherein formula (05) represents a (S)-1-oxo-1-(piperidin-1-yl)propan-2-yl butyrate.

5. Compound synthesized according to claim 1, **characterized by** formula (08): 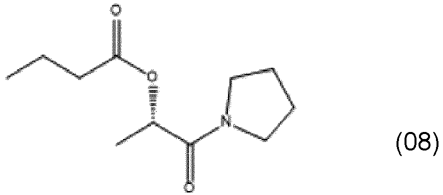 wherein formula (08) represents a (*S*)-1-oxo-1(pyrrolidin-1-yl)propan-2-yl butyrate obtained from enantiomerically pure (*S*)-ethyl lactate.

6. Compound synthesized according to claim 1, **characterized by** formula (10): 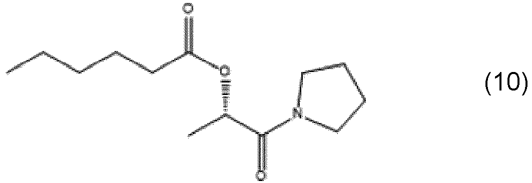 wherein formula (10) represents a (*S*)-1-oxo-1-(pyrrolidin-1-yl)propan-2-yl hexanoate.

7. Compound synthesized according to claim 1, **characterized by** formula (11): 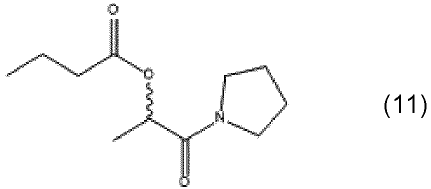 wherein formula (11) represents a racemic mixture of the substance 1-oxo-1(pyrrolidin-1-yl)propan-2-yl.

8. Use of compounds according to any one of the preceding claims, **characterized by** being used as an active ingredient for arthropod repellent composition, in their possible forms, such as lotion, spray, cream gel, combined with sunscreen, aerosol, environmental repellent capsule, liquid for electric sprinkler, microcapsules and/or capsules using the active ingredient as a repellent, repellent powder for indoor use, repellent powder for outdoor use, indoor and/or outdoor repellent gel, repellent wall paint, repellent coating putty, in addition to use in animals and/or agricultural environment.

## Amended claims

Statement under Art. 19.1 PCT
The Applicant advises that claim 1 has been amended in order to emphasize that the repellents in question are derived from lactic acid and its derivatives, and that the hydroxyl functional group and the carboxylic acid functional group present in such substances are transformed into ester and amide, respectively.

It is known that lactic acid, which is found in the perspiration and body odor of warm-blooded animals, interacts with mosquitoes' receptors and, therefore, acts as an attractant for them.

Thus, assuming that some effective repellents known in the literature have amide and ester groups in their structures, the Applicant unexpectedly proposes repellents derived from lactic acid and its derivatives, containing modifications in the irstructure to provide the de sire deffects with low toxicity. Support for the se amendments can be found in paragraphs [016], [029] and [030].

With respect to claim 3, the Applicant understands the objection raised regarding compound (4) and, without prejudice to the understanding and scope of the protection claimed by the present invention, agrees with the removal of this claim.

The other amendments made in claims 2, 4, 5, 6 and 7 were merely for the sake of better defining the term used in the preamble of the claims.

However, in the Written Opinion, an objection was made regarding an alleged lack of description of the figures in the Specification. In this regard, the Applicant respectfully disagrees with such objection and takes the opportunity to inform that all 16 figures contained in the present application are briefly described in the Section "BRIEF DESCRIPTION OFTHE FIGURES", more precisely on pages 9 and 10, paragraphs [041] to [057].
